# EUROPEAN PATENT SPECIFICATION

(11) **EP 1 663 122 B1**
(45) Date of publication and mention of the grant of the patent: **03.03.2010**
(21) Application number: 04755980.2
(22) Date of filing: 23.06.2004
(51) Int. Cl.: A61K 8/42

(54) **MOUTH RINSE COMPOSITIONS CONTAINING N-ACYL-ARGININE ALKYL ESTER SALTS**
MUNDWASSER MIT EINEM N-ACYL-ARGININALKYLESTERSALZ
COMPOSITIONS DE RINCAGE DE BOUCHE CONTENANT DES SELS D'ALKYLESTER N-ACYL-ARGININE

(30) Priority: 23.06.2003 US 601478; 23.06.2004 US 875059
(43) Date of publication of application: 07.06.2006
(73) Proprietor: Colgate-Palmolive Company, New York NY 10022-7499 (US)
(72) Inventor: BOYD, Thomas, J., Metuchen, New Jersey 08840 (US); GAFFAR, Abdul, Princeton, New Jersey 08540 (US); VISCIO, David, B., Monmouth Junction, New Jersey 08852 (US)
(74) Representative: Daniels, Jeffrey Nicholas
(86) International application number: PCT/US2004/020192
(87) International publication number: WO 2005/000261

(56) References cited:
- EP-A- 0 485 616
- US-A- 5 695 745
- US-A- 5 874 068

## Description

### INTRODUCTION

The present invention relates generally to aqueous oral compositions effective in retarding bacterial plaque accumulation on teeth and more particularly to stable aqueous compositions containing an antimicrobial arginine derivative compound.

Dental plaque is a soft deposit which forms on teeth and is comprised of an accumulation of bacteria and bacterial by-products. Plaque adheres tenaciously at the points of irregularity or discontinuity, e.g., on rough calculus surfaces, at the gum line and the like. Besides being unsightly, plaque is implicated in the occurrence of gingivitis and other forms of periodontal disease.

A wide variety of antibacterial agents have been suggested in the art to retard plaque formation and the oral infections associated with plaque formation. For example, U.S. Patent 5,874,068 discloses aqueous oral compositions containing the arginine derivative compound, N^{α}-acyl amino acid ester and salts thereof, as being effective to counter plaque formation by bacterial accumulation in the oral cavity. According to U.S. Patent 5,874,068, as the N^{α}-lauryl-L-arginine alkyl ester is unstable in aqueous environments such as mouthrinses and generally undergoes hydrolysis reactions typical of esters, the arginine derivative compound being stabilized against hydrolysis by the presence in the mouthrinse of a monohydroxy alcohol represented by the formula ROH where R is an alkyl group containing 1 t o 8 carbons, such formula including a monohydroxy alcohol such as ethanol which is present in the mouthrinse at a concentration in the range of about 10 to 35% v/v.

U.S. Patent 5,874,068 further provides for the stability of the mouthrinse having the ROH alcohol by providing an acidic buffer, such as benzoate/benzoic acid, succinate/succinic acid, acetate/acetic acid, or other orally acceptable buffers with dissociation constants within the desired pH range for stability of the N^{α}-lauryl-L-arginine alkyl ester, preferably having a pH of between 3 to 7 and most preferably between 4 to 5.

Other prior art concerned with the antibacterial efficacy of arginine derivative compounds include UK Patent Publication 1352420 which discloses that arginine alkyl ester compounds exhibit antibacterial activity in the oral cavity against bacterium belonging to the genus, Lactobacillus, a main pathogen of dental caries and a bacterium belonging to the genus staphylococcus, a main pathogen of alveolar pyorrhea.

U.S. Patent 5,266,306 discloses an oral composition containing a bactericidal amount of cetylpyridinium chloride and the arginine derivative compound N^{α}-acyl amino acid alkyl ester such as N^{α} -cocoyl-L-arginine methyl ester hydrochloride salt, the arginine derivative compound salt being effective to promote the absorption of cetylpyridinium chloride on tooth surfaces. U.S. Patent 5,695,745 discloses mouthwash formulations which include a citric acid component.

### SUMMARY

In accordance with the present invention there is provided a chemically stable aqueous oral composition comprising:
(a) a safe and effective amount of an antibacterial ester of the formula (I) 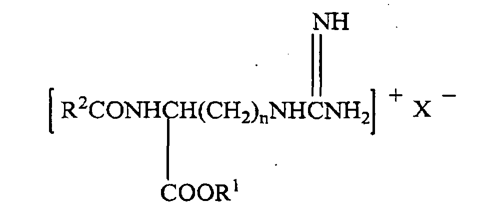 where R¹ is an alkyl chain of 1 to 8 carbon atoms, and R² is an alkyl chain of 6 to 30 carbon atoms, and X is an anion;
(b) a stabilizing surfactant;
(c) a humectant; and
(d) water, wherein the composition does not contain an acidic buffer.
In one embodiment, the composition comprises an alcohol.

In another aspect, the present invention provides compositions comprising:
(a) a safe and effective amount of an antibacterial ester of the formula (I) above
(b) a surfactant;
(c) a humectant; and
(d) water;
wherein said composition is substantially free of monohydric alcohols.

It has been discovered that compositions and methods of this invention afford advantages over stable aqueous mouthwash compositions using antibacterial esters, such as those known in the art including one or more of the following: improved bioavailability and efficacy of an antibacterial ester, potential to reduce pH of a mouth rinse solution employing the antibacterial ester, potential elimination of an acidic buffer and attendant streamlining of manufacturing of the mouth rinse, ability to formulate an alcohol-free mouth rinse, as well as a stable mouth rinse containing alcohols. Further uses, benefits and embodiments of the present invention are apparent from the description set forth herein.

### DESCRIPTION

The following definitions and non-limiting guidelines must be considered in reviewing the description of this invention set forth herein. The headings (such as "Introduction" and "Summary,") and sub-headings (such as "Aqueous Vehicle" "Antibacterial Ester", "Surfactant", "Optional Ingredients", and "Methods") used herein are intended only for general organization of topics within the disclosure of the invention, and are not intended to limit the disclosure of the invention or any aspect thereof. In particular, subject matter disclosed in the "Introduction" may include aspects of technology within the scope of the invention, and may not constitute a recitation of prior art. Subject matter disclosed in the "Summary" is not an exhaustive or complete disclosure of the entire scope of the invention or any embodiments thereof. Classification or discussion of a material within a section of this specification as having a particular utility is made for convenience, and no inference should be drawn that the material must necessarily or solely function in accordance with its classification herein when it is used in any given composition.

The citation of any references herein does not constitute an admission that those references are prior art or have any relevance to the patentability of the invention disclosed herein. Any discussion of the content of references cited in the Introduction is intended merely to provide a general summary of assertions made by the authors of the references, and does not constitute an admission as to the accuracy of the content of such references.

The description and any specific examples, while indicating embodiments of the invention, are intended for purposes of illustration only and are not intended to limit the scope of the invention. Moreover, recitation of multiple embodiments having stated features is not intended to exclude other embodiments having additional features, or other embodiments incorporating different combinations the stated of features. Specific Examples are provided for illustrative purposes of how to make and use the compositions and methods of this invention and, unless explicitly stated otherwise, are not intended to be a representation that given embodiments of this invention have, or have not, been made or tested.

As used herein, the words "preferred" and "preferably" refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As used herein, the word 'include," and its variants, is intended to be non-limiting, such that recitation of items in a list is not to the exclusion of other like items that may also be useful in the materials, compositions, devices, and methods of this invention.

As referred to herein, all compositional percentages are by weight of the total composition, unless otherwise specified.

As referred to herein, the word "substantially," when applied to a characteristic of a composition or method of this invention, indicates that there may be variation in the characteristic without having substantial effect on the chemical or physical attributes of the composition or method.

As used herein, the term "about," when applied to the value for a parameter of a composition or method of this invention, indicates that the calculation or the measurement of the value allows some slight imprecision without having a substantial effect on the chemical or physical attributes of the composition or method. If, for some reason, the imprecision provided by "about" is not otherwise understood in the art with this ordinary meaning, then "about" as used herein indicates a possible variation of up to 5% in the value.

### Aqueous Vehicle

Water can comprise from about 50% to about 80% by weight, preferably from about 55% to about 75% by weight of the aqueous oral compositions of the present invention. These amounts of water include the free water which is added, plus that amount which is introduced with other materials.

The aqueous oral composition of the present invention such as a mouthrinse is prepared using a vehicle which contains water and a humectant. The humectant is generally a mixture of humectants, such as glycerin and sorbitol, and a polyhydric alcohol such as propylene glycol, butylene glycol, hexylene glycol, polyethylene glycol. The humectant content is in the range of about 5 to abut 40% by weight and preferably about 10 to about 30% by weight. The water content is in the range of about 50 to about 80% by weight and preferably 55 to about 75% by weight.

In certain embodiments of the present invention, the mouth rinse vehicle may further comprise a monohydric mouth rinse represented by the general formula R'OH, where R' is an alkyl group having between 1 to 8 carbon atoms. The aqueous oral composition containing a monohydric alcohol is stabilized by the presence of a stabilizing surfactant system, as described in greater detail below. In other embodiments of the present invention, the mouth rinse vehicle may be formulated to be free of a monohydric alcohol. In a preferred embodiment of the present invention, the composition does not comprise an acidic buffer. In various embodiments, the pH of the composition is from about 5 to about 8, optionally from about 6 to about 8, optionally from about 6 to about 7.

### Antibacterial Ester

In preferred embodiments of the present invention an antibacterial ester is represented by the formula 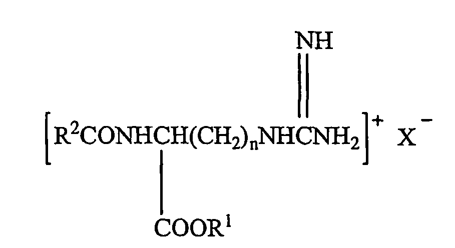 where R¹ is an alkyl chain of 1 to 8 carbon atoms, preferably from 1 t o 3 carbon atoms, and most preferably 3 carbon atoms; R² is an alkyl chain of 6 to 30 carbon atoms, preferably from 10 to 12 carbon atoms, and mixtures thereof; and X is an anion. In various embodiments, the R²CO moiety comprises a natural fatty acid residue such as a natural fatty acid selected from the group consisting of coconut oil fatty acid, tallow fatty acid residue, or a mono-fatty acid residue such as selected from the group consisting of lauroyl (C₁₂), myristyl (C₁₄), stearoyl (C₁₈) fatty acid residues, and mixtures thereof. In one embodiment, the R²CO moiety comprises a lauroyl fatty acid residue.

X may be any counter-anion that provides a reasonable degree of solubility in water (preferably at least about 1g in 1L of water). Examples of X counter anions which form antibacterial ester salts of the above identified formula, include inorganic acid salts, such as those comprising halogen atoms (e.g., chloride or bromide) or dihydrogen phosphate, or an organic salt such as acetate, tautarate, citrate, or pyrrolidone-carboxylate (PCA). The chloride salt is preferred.

Examples of antibacterial ester compounds preferred in the practice of the present invention are antibacterial ester compound of the above-identified formula wherein n in the formula equals 3 useful in the practice of the present invention include N'-cocoyl-L-arginine methyl ester, N^{α}-cocoyl-L-arginine ethyl ester, N^{α}-cocoyl-L-arginine propyl ester, N^{α}-stearoyl-Larginine methyl ester, N^{α}-stearoyl-L-arginine ethyl ester hydrochloride. The term 'cocoyl" is an abbreviation for coconut oil fatty acid residue, and chloride salts of these compounds, these ester compounds and the salts thereof being referred to in this specification as arginine derivative compounds. Arginine derivative compounds and their salts in particular show excellent inhibitory effect against microorganisms which possess relatively strong resistance to bacteria such as *S*. *aurous, S. mutans, F. nucleatum* which are involved in plaque formation on teeth. An arginine derivative compound preferred in the practice of the invention is the hydrogen chloride salt of ethyl lauroyl arginine.

The antibacterial ester of the present invention is present in the aqueous oral compositions at a concentration of about 0.05 to about 2.0% by weight and preferably about 0.075 to about 1% by weight.

### Surfactant

Surfactants useful in the practice of the present invention include nonionic and zwitterionic surfactants. Suitable nonionic surfactants useful in the present invention include poly(oxyethylene)-poly(oxypropylene) block copolymers. Such copolymers are known commercially by the non-proprietary name of poloxamers, which name is used in conjunction with a numeric suffix to designate the individual identification of each copolymer. Poloxamers may have varying contents of ethylene oxide and propylene oxide which results in poloxamers which have a wide range of chemical structures and molecular weights.

A preferred group of nonionic surfactants useful in the present invention include condensates of sorbitan esters of fatty acids with ethylene oxide (polysorbates) such as sorbitan monooleate with from about 20 to about 60 moles of ethylene oxide (e.g., "Tweens", a trademark of ICI US, Inc.). Particularly preferred polysorbates are Polysorbate 20 (polyoxyethylene 20 sorbitan monolaurate, Tween 20) and Polysorbate 80 (polyoxyethylene 20 sorbitan monooleate, Tween 80).

In certain preferred embodiments of the present invention, the antibacterial N^{α}-acyl amino acid alkyl ester is stabilized in the aqueous solution by the surfactant system (i.e., a stabilizing surfactant system). By stabilizing surfactant system, it is meant that a surfactant is included that is maintains the bioavailability of the N^{α}-acyl amino acid alkyl ester antibacterial ingredient, which is generally believed to be by substantially preventing the hydrolysis of the N^{α}-acyl amino acid alkyl ester, without the need for additional ingredients, such as acidic buffers. Thus, in certain preferred embodiments of the present invention, the aqueous composition comprises a stabilized surfactant system comprising zwitterionic surfactants, most particularly betaine surfactants have been found to stabilize the antibacterial N^{α}-acyl amino acid alkyl ester without the inclusion of an acidic buffer, thereby maintaining the bioavailability of the antibacterial ester. Thus, the zwitterion surfactants useful in certain embodiments of the present invention, particularly betaine surfactants, include surfactants disclosed in U.S. Patent 5,180,577. Typical alkyldimethyl betaines include decyl betaine 2-(N-decyl-N,N-dimethylammonio) acetate, cocobetaine or 2-(N-coc-N, N-dimethyl ammonio) acetate, myristyl betaine, palmityl betaine, lauryl betaine, cetyl betaine, stearyl betaine, etc. The amidobetaines are exemplified by cocoamidoethyl betaine, cocoamidopropyl betaine, lauramidopropyl betaine and the like. The preferred betaine is the cocoamidopropyl betaine.

The surfactant is present in the aqueous oral compositions of the present invention range from about 0.1% to about 5% by weight preferably from about 0.6% to about 2.0% by weight.

### Optional Ingredients

Any suitable flavoring or sweetening material may also be incorporated in the mouthrinse composition of the present invention. Examples of suitable flavoring constituents are flavoring oils, e.g., oils of spearmint, peppermint, wintergreen, sassafras, clove, sage, eucalyptus, marjoram, cinnamon, lemon and orange and methyl salicylate. Suitable sweetening agents include sucrose, lactose, maltose, sorbitol, xylitol, sodium cyclamate, perillartine and sodium saccharin. Suitably, flavor and sweetening agents may together comprise from 0.0 1% to 5% by weight or more of the mouthrinse composition and at such concentrations render the mouthrinse with a palatability acceptable to the user.

The oral composition of the present invention may additionally contain a fluoridating agent to aid in preventing dental caries as well as antibacterial metal salts, halogenated diphenyl ethers and enzymes. Fluoridating agents suitable for use in the oral compositions of the present invention includes sodium fluoride, potassium fluoride, stannous fluoride and complex fluorides such as sodium monofluorophosphate. The fluoridating agent is most desirably present in an amount to provide 1000-2000 ppm fluoride ion in the composition.

Examples of antibacterial metal salts suitable for use in the present invention include stannous salts such as stannous chloride, stannous gluconate, zinc salts such as zinc chloride, zinc gluconate, zinc citrate and copper salts such as copper gluconate. Examples of halogenated diphenyl ethers include Triclosan and enzymes include papain and glycoamylase. These agents may be present in the composition of the present invention at concentrations of about 0.1 to about 2% by weight.

Antitartar agents compatible with antibacterial esters such as ethyl lauroyl arginine may also be included in the oral composition of the present invention. An example of such antitartar agents include cationic polyphosphonates such as water soluble quaternary amino alkylene phosphonic compounds as disclosed in U.S. Patent 4,118,472. These antitartar agents may be included in the oral composition of the present invention at a concentration of about 0.1 to about 5% by weight.

Antitartar agents which are not compatible with antibacterial esters such as pyrophosphate and polyphosphate salts may be included in one component of a dual component oral composition system in which a first component contains the antibacterial ester and the second component contains the incompatible antitartar salt, the first and second components being maintained separate from each other until dispersed and combined for application to the teeth.

### Methods

The present invention further provides a method of making a stable aqueous antiplaque oral composition comprising admixing a safe and effective amount of an antibacterial ester represented by the formula 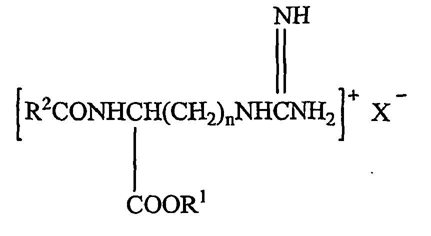 where R¹ is an alkyl chain of 1 to 8 carbon atoms, and R² is an alkyl chain of 6 to 30 carbon atoms and X is an anion. The antibacterial is admixed with a stabilizing surfactant system, a humectant, and water to form an aqueous composition. In certain embodiments, the oral composition is substantially free of an acidic buffer.

The following examples further describe and demonstrate embodiments within the scope of the present invention.

### Example 1

A mouth rinse of the present invention having a pH of 5.0 is prepared by dissolving in water each of the ingredients listed in Table I below with agitation in a glass mixing vessel.

**TABLE I**

| **Ingredient** | **Wt. %** |
|---|---|
| Ethyl Lauroyl arginate HCI (ELAH) | 0.1 |
| Sorbitol | 10.0 |
| Glycerin | 10.0 |
| Propylene glycol | 7.0 |
| Polysorbate 20 | 0.8 |
| Cocoamidopropyl betaine | 0.8 |
| Sodium saccharin | 0.03 |
| Flavor | 0.10 |
| Water | Q.S. |

After 9 months at room temperature, the ELAH concentration is determined by Gas Chromatography - Mass Spectrometry to be unchanged at 0.1 % by weight.

### Example 2

A mouth rinse of the present invention is prepared by dissolving in water each of the ingredients listed in Table II below with agitation in a glass mixing vessel. The Ethyl Lauroyl Arginate HCl (ELA) can be included in concentrations of 0.1, 0.2, or 0.3 weight percent.

**TABLE II**

| **Ingredient** | **Wt. %** |
|---|---|
| Ethyl Lauroyl arginate HCI (ELAH) | 0.1 or 0.2 or 0.3 |
| Ethyl Alcohol | 15.0 |
| Sorbitol | 10.0 |
| Glycerin | 10.0 |
| Propylene glycol | 7.0 |
| Cocoamidopropyl betaine | 0.6 |
| Sodium saccharin | 0.03 |
| Flavor | 0.10 |
| Water | Q.S. |

The description of the invention and examples provided herein is merely exemplary in nature and, thus, variations that do not depart from the gist of the invention are intended to be within the scope of the invention. Such variations are not to be regarded as a departure from the scope of the invention.

## Claims

1. A stable aqueous antiplaque oral composition comprising:
(a) a safe and effective amount of antibacterial ester of the formula 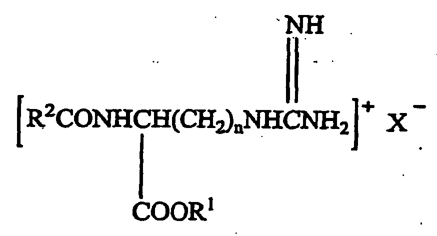 where R¹ is an alkyl chain of 1 to 8 carbon atoms, and R² is an alkyl chain of 6 to 30 carbon atoms, and X is an anion;
(b) a stabilizing surfactant;
(c) a humectant; and
(d) water; said composition not comprising an acidic buffer.

2. A composition according to claim 1, wherein n is equal to 3.

3. The composition according to claim 2, wherein said antibacterial ester comprises a hydrochloride salt of ethyl lauroyl arginine.

4. The composition according to claim 1, wherein the concentration of said antibacterial ester compound is between 0.02% to 2% by weight.

5. A composition according to claim 1, wherein said stabilizing surfactant comprises a zwitterionic surfactant

6. A composition according to claim 5, wherein said zwitterionic surfactant comprises a betaine surfactant.

7. A composition according to claim 5, wherein said stabilizing surfactant comprises a zwitterionic surfactant and a nonionic surfactant.

8. A composition according to claim 1, further comprising an alcohol.

9. A composition according to claim 8, wherein said alcohol is monohydric and represented by the formula R'OH, where R' is an alkyl group having between 1 to 8 carbon atoms.

10. A composition according to claim 8, wherein said alcohol is polyhydric.

11. A composition according to claim 10, wherein said polyhydric alcohol comprises propylene glycol.

12. A composition according to claim 1, wherein the composition is substantially free of monohydric alcohol.

13. A composition according to claim 1, wherein said humectant comprises a mixture of polyhydric alcohols.

14. A composition according to claim 1, having a pH of from 6 to 8.

## Patentansprüche

1. Stabile, wässrige, orale Antiplaquezusammensetzung, umfassend:
a) eine sichere und effektive Menge eines antibakteriellen Esters der Formel 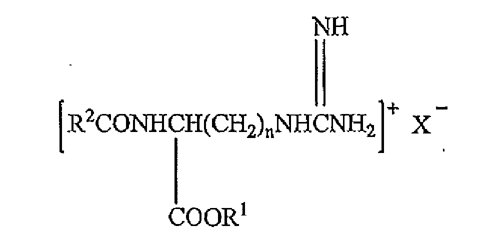 wobei R¹ eine Alkylkette von 1 bis 8 Kohlenstoffatomen und R² eine Alkylektte von 6 bis 30 Kohlenstoffatomen und X ein Aninon ist,
b) ein stabilisierendes Tensid,
c) ein Feuchthaltemittel, und
d) Wasser, wobei die Zusammensetzung keinen sauren Puffer enthält.

2. Zusammensetzung nach Anspruch 1, wobei n gleich 3 ist.

3. Zusammensetzung nach Anspruch 2, wobei der antibakterielle Ester ein Hydrochloridsalz von Ethyllaurylarginin umfasst.

4. Zusammensetzung nach Anspruch 1, wobei die Konzentration der antibakteriellen Esterverbindung zwischen 0,02 Gew.-% und 2 Gew.-% liegt.

5. Zusammensetzung nach Anspruch 1, wobei das stabilisierende Tensid ein Zwitterion-Tensid umfasst.

6. Zusammensetzung nach Anspruch 5, wobei das Zwitterion-Tensid ein Betain-Tensid umfasst.

7. Zusammensetzung nach Anspruch 5, wobei das stabilisierende Tensid ein Zwitterion-Tensid und ein nicht-ionisches Tensid umfasst.

8. Zusammensetzung nach Anspruch 1, die weiterhin Alkohol umfasst.

9. Zusammensetzung nach Anspruch 8, wobei der Alkohol einwertig ist und durch die Formel R'OH wiedergegeben wird, worin R' eine Alkylgruppe mit zwischen 1 bis 8 Kohlenstoffatomen ist.

10. Zusammensetzung nach Anspruch 8, wobei der Alkohol mehrwertig ist

11. Zusammensetzung nach Anspruch 10, wobei der mehrwertige Alkohol Propylenglycol umfasst.

12. Zusammensetzung nach Anspruch 1, wobei die Zusammensetzung im Wesentlichen frei von einwertigem Alkohol ist.

13. Zusammensetzung nach Anspruch 1, wobei das Feuchthaltemittel eine Mischung von mehrwertigen Alkoholen umfasst.

14. Zusammensetzung nach Anspruch 1, die einen pH-Wert von 6 bis 8 aufweist.

## Revendications

1. Composition orale antiplaque aqueuse stable comprenant :
(a) une quantité sûre et efficace d'un ester antibactérien de formule 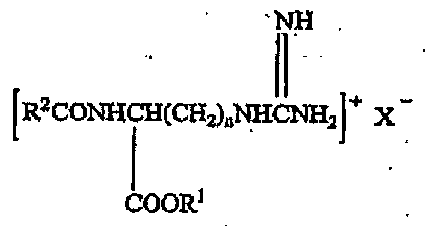 où R¹ est une chaîne alkyle de 1 à 8 atomes de carbone, et R² est une chaîne alkyle de 6 à 30 atomes de carbone, et X est un anion ;
(b) un tensioactif de stabilisation ;
(c) un humectant ; et
(d) de l'eau ; ladite composition ne comprenant pas de tampon acide.

2. Composition selon la revendication 1, où n est égal à 3.

3. Composition selon la revendication 2, où l'ester antibactérien comprend un sel chlorhydrate d'éthyl lauroyl arginine.

4. Composition selon la revendication 1, où la concentration dudit composé ester antibactérien est comprise entre 0,02 % à 2 % en poids.

5. Composition selon la revendication 1, où ledit tensioactif de stabilisation comprend un tensioactif zwitterionique.

6. Composition selon la revendication 5, où ledit tensioactif zwitterionique comprend un tensioactif bétaïne.

7. Composition selon la revendication 5, où ledit tensioactif de stabilisation comprend un tensioactif zwitterionique et un tensioactif non ionique.

8. Composition selon la revendication 1, comprenant en outre un alcool.

9. Composition selon la revendication 8, où ledit alcool est monohydroxylé et représenté par la formule R'OH, où R' est un groupe alkyle ayant entre 1 à 8 atomes de carbone.

10. Composition selon la revendication 8, où ledit alcool est polyhydroxylé.

11. Composition selon la revendication 10, où ledit alcool polyhydroxylé comprend du propylène glycol.

12. Composition selon la revendication 1, où la composition est sensiblement exempte d'alcool monohydroxylé.

13. Composition selon la revendication 1, où ledit humectant comprend un mélange d'alcools polyhydroxylés.

14. Composition selon la revendication 1, ayant un pH de 6 à 8.
